# EUROPEAN PATENT APPLICATION

(11) **EP 4 446 729 A2**
(43) Date of publication of application: **16.10.2024**
(21) Application number: 24162870.0
(22) Date of filing: 12.03.2024
(51) Int. Cl.: G01N 21/39, G01N 21/53

(54) **APPARATUS AND METHOD FOR SENSING GAS AND PARTICULATE MATTER USING AN OPTICAL BEAM**

(30) Priority: 13.04.2023 US 202318300252
(71) Applicant: Honeywell International Inc., Charlotte, NC 28202 (US)
(72) Inventor: BROWN, Andy Walker, Charlotte, 28202 (US); BORK, Stephan, Charlotte, 28202 (US)
(74) Representative: Haseltine Lake Kempner LLP

(57) **Abstract**

Various embodiments are directed to methods, apparatuses, and systems for sensing one or more gases and particulate matter. In various embodiments, a method of detecting a gas and particulate matter within a sensing region comprises emitting, from a beam component, an optical beam along a beam path defined within a sensing region; detecting particulate matter within the sensing region based on particulate data generated by one or more photodetector, the particulate data being defined by a detection of a scattered portion of the optical beam reflected from a particulate positioned along the beam path; and detecting a first gas based on gas data generated by the beam component, wherein the gas data is generated by the beam component via an optical gas sensing means, and wherein the gas data is defined by a detected absorption of the optical beam at a first wavelength corresponding to the first gas.

## Description

### FIELD OF THE INVENTION

Example embodiments of the present disclosure relate generally to monitoring air quality within an environment and, more particularly, to apparatuses and methods for detecting and characterizing gases and particulate matter within a designated environment.

### BACKGROUND

Applicant has identified many technical challenges and difficulties associated with simultaneously detecting gases and particulate matter within the same environment. For example, many methods and systems fail to allow for a singular sensing device to detect both a gas concentration and a particulate matter concentration within the common environment.

### BRIEF SUMMARY

Various embodiments described herein relate to methods, apparatuses, and systems for sensing one or more gases and particulate matter. In accordance with various embodiments of the present disclosure, a method of detecting one or more gases and particulate matter within a sensing region may comprise emitting, from a beam component, an optical beam along a beam path defined at least partially within a sensing region; detecting particulate matter within the sensing region based at least in part on particulate data generated by one or more photodetector, the particulate data being defined at least in part by a detection of a scattered portion of the optical beam reflected from a particulate positioned along the beam path; and detecting a first gas within the sensing region based at least in part on gas data generated by the beam component, wherein the gas data is generated by the beam component via an optical gas sensing means, and wherein the gas data is defined at least in part by a detected absorption of at least a portion of the optical beam at a first wavelength corresponding to the first gas.

In various embodiments, the optical beam may embody an active optical beam that is pulsed from the beam component such that the active optical beam defines an optical pulse rate. In certain embodiments, the method may further comprise executing a data processing operation wherein a photodetector signal defined at least in part by the particulate data generated by the one or more photodetectors is processed with the optical pulse rate using a phase-locked loop to enhance the photodetector signal by at least partially reducing signal noise. In various embodiments, the optical gas sensing means may be a dual-frequency comb spectroscopy operation. In certain embodiments, the gas data generated by the beam component may comprise a frequency-comb data stream including at least a first wavelength and a second wavelength. In various embodiments, the method may further comprise providing a color filter relative to the one or more photodetectors such that the scattered portion of the optical beam is passed through the color filter.

In various embodiments, the method may further comprise transmitting the particulate data generated by the one or more photodetector to a controller, and determining, via the controller, a particulate matter concentration associated with the particulate matter based at least in part on the particulate data. In various embodiments, the method may further comprise transmitting the gas data generated by the beam component to a controller, and determining, via the controller, a gas concentration associated with the first gas based at least in part on the gas data. In certain embodiments, the gas data may be further defined by an absorption intensity defined at the first wavelength, and wherein the gas concentration associated with the first gas is determined based at least in part on the absorption intensity defined at least at the first wavelength.

In various embodiments, the one or more photodetectors may define a plurality of photodetectors, and wherein the method further comprises determining a cumulative particulate matter concentration based on the respective particulate data generated by each of the plurality of photodetectors. In certain embodiments, the beam path defined by the optical beam may define a looped path configuration, wherein the beam path is defined along two or more distinct linear axes. In certain embodiments, the beam path defined by the optical beam may define a retro-reflection path configuration, wherein the beam path is defined along a single axis between the beam component and a reflective element configured to reflect the optical beam towards the beam component. Further, in certain embodiments, the optical beam may be emitted in an emission direction at least substantially towards a mobile reflection platform configured for movement relative to the beam component such that the beam path defines a dynamic configuration. Further still, in certain embodiments, the method may further comprise detecting a movement of the mobile reflection platform from a first position to a second position relative to the beam component; and at least partially adjusting the emission direction defined by the beam path such that at least a portion of the beam path is defined along an axis oriented between the beam component and the second position of the mobile reflection platform.

In various embodiments, a sensor configured for detecting one or more gases and particulate matter may comprise a beam component configured to emit an optical beam along a beam path defined at least partially within a sensing region of a sensor, wherein the beam component is further configured to generate gas data associated with a first gas positioned the sensing region via an optical gas sensing means; one or more photodetector configured to generate particulate data defined at least in part by a detection of a scattered portion of the optical beam reflected off a particulate positioned along the beam path; and a controller configured to detect particulate matter within sensing region based at least in part on the particulate data generated by the one or more photodetector, and detect the first gas based at least in part on the gas data generated by the beam component, wherein the gas data is defined at least in part by a detected absorption of at least a portion of the optical beam at a first wavelength corresponding to the first gas.

In various embodiments, the optical beam may embody an active optical beam that is pulsed from the beam component such that the active optical beam defines an optical pulse rate. In certain embodiments, the controller may be further configured to execute a data processing operation wherein a photodetector signal defined at least in part by the particulate data generated by the one or more photodetectors is processed with the optical pulse rate using a phase-locked loop to enhance the photodetector signal by at least partially reducing signal noise. In various embodiments, the optical gas sensing means may be a dual-frequency comb spectroscopy operation. In certain embodiments, the gas data generated by the beam component may comprise a frequency-comb data stream including at least a first wavelength and a second wavelength. In various embodiments, the one or more photodetectors may define a plurality of photodetectors each oriented to face in a direction at least substantially towards a respective portion of the beam path and configured to generate respective particulate data defined at least in part by respective detections of scattered optical beam light reflected from one or more particulates positioned at the respective portion of the beam path adjacent the respective photodetector.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 illustrates a schematic view of an exemplary gas sensor in accordance with various embodiments of the present disclosure;
FIG. 2 illustrates a schematic view of an exemplary apparatus in accordance with various embodiments of the present disclosure;
FIG. 3 is an illustrative flowchart of various steps for an example method in accordance with various embodiments of the present disclosure;
FIG. 4 illustrates a schematic view of an exemplary gas sensor in accordance with various embodiments of the present disclosure; and
FIG. 5 illustrates a schematic view of an exemplary gas sensor in accordance with various embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from one of these components.

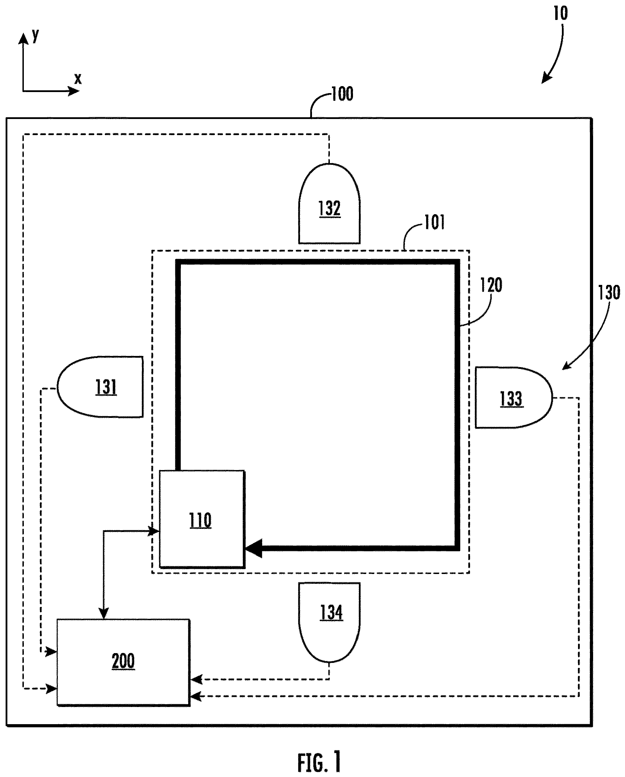

FIG. 1 illustrates a schematic view of an exemplary gas sensor in accordance with various embodiments of the present disclosure. In particular, FIG. 1 illustrates an exemplary sensor 10 configured to execute a gas sensing operation to detect one or more gases within the sensor 10 (e.g., within a sensing region 101) and a particulate matter (PM) sensing operation to detect particulate matter within the sensor 10 (e.g., within a sensing region 101). For example, in various embodiments, the exemplary sensor may comprise one or more sensing systems configured to capture data associated with a volume of fluid (e.g., air, gas, and/or a combination thereof) received by the sensor such that the sensor 10 is operable to at least substantially simultaneously sense a volume of gas and one or more particulates present within the sensor 10 (e.g., within the sensor housing 100).

In various embodiments, an exemplary sensor 10 may comprise a sensor housing 100 embodying an exterior shell having an interior housing portion defined therein, within which the sensing region 101 of the sensor 10 may be defined and at least a portion of the one or more sensing systems may be disposed. Further, in various embodiments, the sensor 10 may comprise a beam component 110 configured to emit a light beam into the sensing region 101 such that a beam path 120 along which the beam travels is defined within the sensing region 101, and one or more photodetector 130 configured to capture a scattered portion of the beam defined by light that is scattered and/or emitted from (e.g., reflected off of) particulate matter positioned along the beam path 120. In various embodiments, the exemplary sensor 10 may further comprise a controller 200 communicatively connected to the beam component 110 and the one or more photodetector 130 such that the respective data captured by each of the beam component 110 and the one or more photodetector 130 may be transmitted (e.g., via a wired and/or wireless communication) to the controller 200 for further processing as part of one or more of a gas sensing operation and a particulate matter sensing operation being executed by the sensor 10.

In various embodiments, the beam component 110 may be configured to generate a light beam (e.g., ultraviolet, visible, infrared, or multiple color light) that is selectively emitted into the sensing region 101 (e.g., a reflection chamber) of the sensor 10 to enable the gas sensing and particulate sensing functionalities of the sensor 10, as described herein. The beam component 110 of an exemplary sensor 10 may comprise a light beam generator and a beam detector component (e.g., a receiver) configured to detect at least a portion of the beam emitted from the light beam generator upon the beam having been reflected at one or more locations (e.g., surfaces) within the sensing region 101 (e.g., a reflection chamber) such that an end portion of the beam path 120 is oriented towards the detector of the beam component 110. For example, the beam component 110 (e.g., the light beam generator) may be configured to emit an active optical beam that propagates through the sensing region 101 and is retro-reflected back to the beam component 110 (e.g., the detector).

In various embodiments, the beam component 110 (e.g., the light beam generator) may be configured to emit an at least substantially non-continuous light beam such that the optical beam embodies an active optical beam that is pulsed along the beam path 120. For example, in such an exemplary configuration, the active optical beam emitted from the beam component 110 may be defined at least in part by an optical pulse rate. As a non-limiting example provided for illustrative purposes, in various embodiments, the optical pulse rate defined by the active optical beam emitted from the beam component 110 may be at least approximately between 1.0 mHz and 10 GHz (e.g., between 1.0 Hz and 100.0 MHz).

In various embodiments, the sensor 10 may be configured such that the optical beam emitted from the beam component 110 propagates through the sensing region 101 of the sensor 10 and is reflected back to the beam component 110. Upon the optical beam being propagated through the sensing region 101 along the beam path 120, the beam component 110 (e.g., a detector thereof) may be configured to detect at least a portion of the optical beam such that the optical absorption intensity at one or more wavelengths defined within the absorption band of one or more designated gases may be determined. In various embodiments, the sensor 10 may be configured to execute a gas sensing operation by using the beam component 110 to carry out an optical sensing means of detecting a volume of gas within the sensing region 101 of the sensor 10, as described herein. For example, the sensor 10 (e.g., the controller 200) may be configured to detect the presence of one or more gases (e.g., a plurality of designated gases) and/or one or more characteristics defined by the one or more gases encountered along the beam path 120 (e.g., within the sensing region 101), such as, for example, one or more gas concentrations, using dual-frequency comb spectroscopy. For example, in various embodiments, the sensor 10 may be configured to execute a gas sensing operation by using the beam component 110 to facilitate a dual-frequency comb spectroscopy operation to detect the presence of a first gas having a first absorption band and/or a second gas having a second absorption band by measuring a first optical absorption intensity defined by the optical beam at a first wavelength within the first absorption band and a second optical absorption intensity defined by the optical beam at a second wavelength within the second absorption band, respectively. In such an exemplary circumstance, the first and second optical absorption intensities may define gas data that may be used by the sensor 10 (e.g., the controller 200) to determine a first gas concentration of the first gas and a second gas concentration of the second gas within the sensor 10. For example, in such an exemplary means of utilizing an active optical beam to detect a designated gas, the gas concentration of the designated gas may be measured based on the optical absorption intensity at a specific wavelength corresponding to that specific gas, which is proportional to the gas concentration defined by the volume of gas. In various embodiments, the beam component 110 may be configured to capture gas data defined at least in part by a frequency-comb data stream and transmit the captured gas data to the controller 200 of the sensor 10.

In various embodiments, the exemplary sensor 10 may be configured to facilitate the processing of one or more specific signals transmitted to the controller 200, such as, for example, a photodetector signal generated by a photodetector 130, by arranging one or more color filters between the photodetector 130 and an adjacent portion of the beam path 120 such that a scattered portion of the optical beam reflected from a particulate positioned along the beam path 120 is passed through the one or more color filters before reaching the photodetector 130. For example, a color filter positioned in front of a photodetector 130 may have various known characteristics that at least partially affect the scattered and/or ambient light detected by the photodetector 130 and, therefore, have a known effect on the photodetector signal that is generated by the photodetector 130. In various embodiments, a color filter positioned in front of a photodetector 130 may facilitate a more accurate detection of a scattered portion of the optical beam by being configured to reject ambient (e.g., background) light present within the sensing region while allowing scattered light that matches one or more of the wavelengths defined by the optical beam to pass therethrough. As such, the one or more color filters may be configured such that the light detected by the photodetectors 130 may be limited to the desired scattered portion of the optical beam.

Further, in various embodiments, as part of the particulate matter sensing operation executed by the sensor 10, the controller 200 may be configured to identify a received photodetector signal as having been transmitted from a specific one of the one or more photodetectors 130 based at least in part on one or more characteristics of the photodetector signal that are identified as corresponding to the color filter placed in front of the photodetector 130. Additionally and/or alternatively, in various embodiments, the one or more color filters through which a detected scattered portion of the optical beam may pass through prior to being detected by the photodetector 130 may cause the resultant photodetector signal (e.g., particulate data) to be defined by one or more signal characteristics, such as, for example, a specific wavelength, that may be used by the controller 200 to enhance and/or at least partially isolate the photodetector signal received from the photodetector 130. In various embodiments wherein the sensor 10 comprises a plurality of photodetectors 130, a corresponding plurality of color filters may be utilized such that a color filter of a distinct color and/or optical properties is placed in front of each of the respective photodetectors 130. Alternatively and/or additionally, in various embodiments, wherein the sensor 10 comprises a plurality of photodetectors 130, the corresponding plurality of color filters of the same color and/or optical properties may be placed in front of each of the respective photodetectors 130.

In various embodiments, the one or more photodetectors 130 may embody a photodiode comprising one or more lens, such as, for example, a condenser lenses, configured to collect a quantity of light that is sufficient to enable the sensor 10 to generate particulate data sufficient for execution of a particulate matter sensing operation. Further, in various embodiments, the one or more photodetectors 130 may defined one or more apertures configured to spatially restrict the light (e.g., ambient light and/or scattered light) detected by the photodetector 130 in order to at least partially restrict the field of view of the photodetector 130 to only an adjacent portion of the sensing region that is occupied by the optical beam, thereby operably minimizing the impact of detected ambient light on the resultant particulate data.

In various embodiments, as illustrated in FIG. 1, the one or more photodetectors 130 of the exemplary sensor 10 may arranged at least substantially proximate a respective portion of the beam path 120 defined by the beam emitted from the beam component 110 and/or oriented to face in a direction at least partially towards the beam path 120 such that the photodetector 130 may detect light scattered and/or emitted from particulate matter (e.g., one or more particulates) positioned within the beam path 120. In various embodiments, the sensor 10 may be configured to execute a particulate matter sensing operation by using the one or more photodetectors 130 to detect particulates within the sensing region 101 and, based on the particulate data generated by the photodetectors 130, determine one or more characteristics of the particulate matter present within the sensing region 101, such as, for example, particulate matter concentration. For example, each of the one or more photodetectors 130 may define a pulse detecting component configured to use laser-based light scattering particle sensing means in order to detect the presence and/or characteristics of one or more particulates within a volume of gas present within the sensor 10. In various embodiments, each of the one or more photodetectors 130 is configured to capture respective particulate data defined at least in part by light pulse data corresponding to one or more scattered portions of the optical beam that may be defined at least in part by a pulse quantity and/or a pulse intensity. Each photodetector 130 may be communicatively coupled with the controller 200 of the sensor 10 such that the particulate data generated by each photodetector 130 may transmitted to the controller 200 as part of a particulate sensing operation. In such an exemplary means of utilizing one or more detected scattered portions of the optical beam to detect particulate matter within the sensing region 101, the particulate matter concentration defined by the one or particulates detected within the sensing region 101 be measured based at least in part on the pulse quantity and/or a pulse intensity defined by the one or more detected particulates.

In various embodiments, the sensor 10 (e.g., the controller 200) may be configured to execute a data processing operation wherein a photodetector signal received from the one or more photodetectors 130 and defined at least in part by the particulate data generated by the one or more photodetectors 130 is used together with a known optical pulse rate defined by an operation of the beam component 110 (e.g., the light beam generator) in a phase-locked loop (PLL) to enhance the photodetector signal. For example, particulate data generated by one or more photodetector 130 of the sensor 10 may be received by the controller 200 and processed with the optical pulse rate using a phase-locked loop to at least partially reduce and/or minimize the background noise within the photodetector signal captured by the photodetector 130.

In various embodiments, as illustrated in FIG. 1, the sensor 10 may comprise a plurality of photodetectors oriented to face a respective portion (e.g., beam segment) of the beam path 120 such that each of the plurality of photodetectors is configured to detect a respective scattered portion of the optical beam reflected off particulate matter present within the respective portion of the beam path 120 at least substantially proximate to the photodetector. As a non-limiting example, the exemplary sensor 10 illustrated in FIG. 1 comprises a plurality of photodetectors including a first photodetector 131, a second photodetector 132, a third photodetector 133, and a fourth photodetector 134, each of which are communicative connected to the controller 200 such that particulate data captured by the respective photodetectors may be individually transmitted to the controller 200. In such an exemplary configuration, the plurality of photodetectors 131, 132, 133, 134 are spatially distributed along the beam path 120. Based at least in part on the particulate data generated by each of the photodetectors 131, 132, 133, 134, the sensor 10 (e.g., the controller 200) may be configured to determine a cumulative particulate matter concentration and/or more accurately spatially resolve the particulate matter concentration within the sensing region 101.

As illustrated in FIG. 1, the exemplary sensor 10 is configured such that the beam propagated from the beam component 110 defines a beam path 120 having a looped path configuration. For example, in such an exemplary configuration, the beam path 120 defined by the optical beam is defined along two or more distinct linear axes, such as, for example, a first axes oriented in an x-direction and a second axes oriented in a y-direction, as defined in the exemplary orientation of the embodiment illustrated in FIG. 1. As illustrated, a beam path 120 having a looped path configuration is defined by at least three (e.g., four) path segments oriented along respective axes that are collectively configured such that the optical beam emitted from the beam component 110, upon being propagated through the sensing region 101 of the sensor 10, is reflected back to the beam component 110.

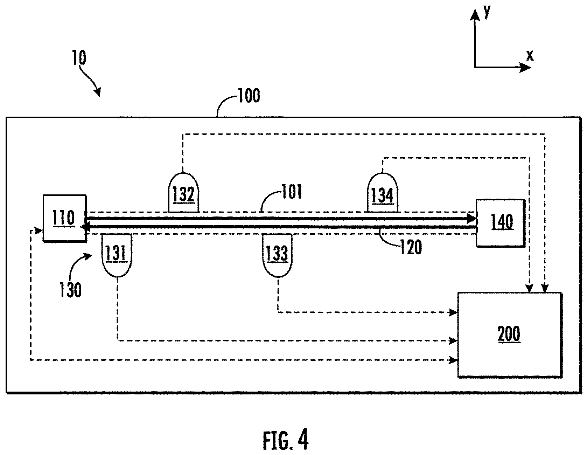

Further, in various embodiments, the 10 may be configured such that the beam emitted from the 110 may define a 120 having a retro-reflection configuration. For example, FIG. 4 illustrates a schematic view of an exemplary sensor 10 in accordance with various embodiments described herein. In particular, the exemplary sensor 10 illustrated in FIG. 4 is configured such that the beam propagated from the beam component 110 defines a beam path 120 having a retro-reflected path configuration. For example, in such an exemplary configuration, the beam path 120 defined by the optical beam is at least approximately oriented along a single axis (e.g., along an x-axis as defined in the exemplary orientation of the embodiment illustrated in FIG. 4) defined between the beam component 110 and a reflective element 140, such as, for example, a corner cube, configured to reflect the optical beam towards the beam component 110. As illustrated, in such an exemplary configuration, each of the one or more photodetector 130 of the sensor 10 may be arranged along the beam path 120 such that the particulate data generated by the one or more photodetectors 130 enables the sensor 10 to more accurately spatially resolve the particulate matter concentration along the sensing region 101 defined between the beam component 110 and the reflective element 140.

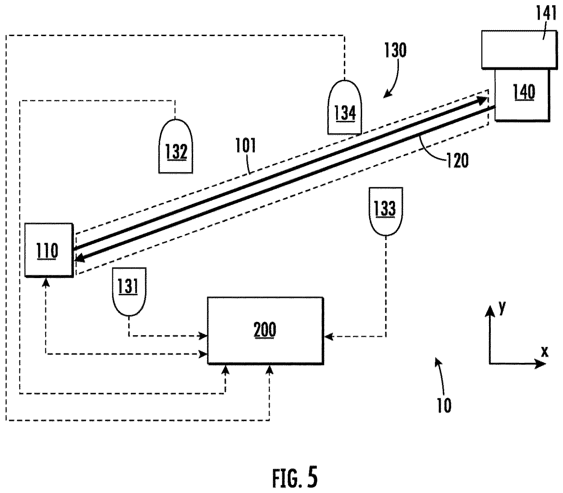

Further still, in various embodiments, the sensor 10 may be configured such that the active optical beam emitted from the beam component 110 may define a beam path 120 having an at least substantially dynamic configuration. For example, FIG. 5 illustrates a schematic view of an exemplary sensor 10 in accordance with various embodiments described herein. In particular, the exemplary sensor 10 illustrated in FIG. 5 is configured such that the beam component 110 is configured to propagate the active optical beam in an emission direction towards a mobile reflection platform configured for movement relative to the beam component 110 such that the orientation of the beam path 120 may be selectively varied over time. For example, a mobile reflection platform may be defined by a reflective element 140, such as, for example, a corner cube, that is secured relative to a mobile platform 141 (e.g., a UAV) configured to move relative to a stationary base platform and/or the one or more photodetectors 130. In such an exemplary configuration, the beam path 120 defined by the optical beam is at least approximately oriented along a single axis defined between the beam component 110 and a reflective element 140, such as, for example, a corner cube, configured to reflect the optical beam back towards the beam component 110. As illustrated, in such an exemplary configuration, each of the one or more photodetector 130 of the sensor 10 may be fixedly secured and/or spatially distributed within an environment such that the particulate data generated by the one or more photodetectors 130 may enable the sensor 10 to more accurately spatially resolve the particulate matter concentration within a sensing region 101 as the mobile platform 141 to which the reflective element 140 is secured is moved between one or more locations. For example, in various embodiments the mobile platform 141 may be controlled such that the position of the reflective element is moved from a first position to a second position relative to the beam component 110. In response, upon detecting the movement of the reflective element 140, the beam component 110 may be configured such that the emission direction defined by the beam path 120 may be at least partially adjusted such that at least a portion of the beam path 120 is defined along an axis oriented between the beam component 110 and the second location of the reflective element 140.

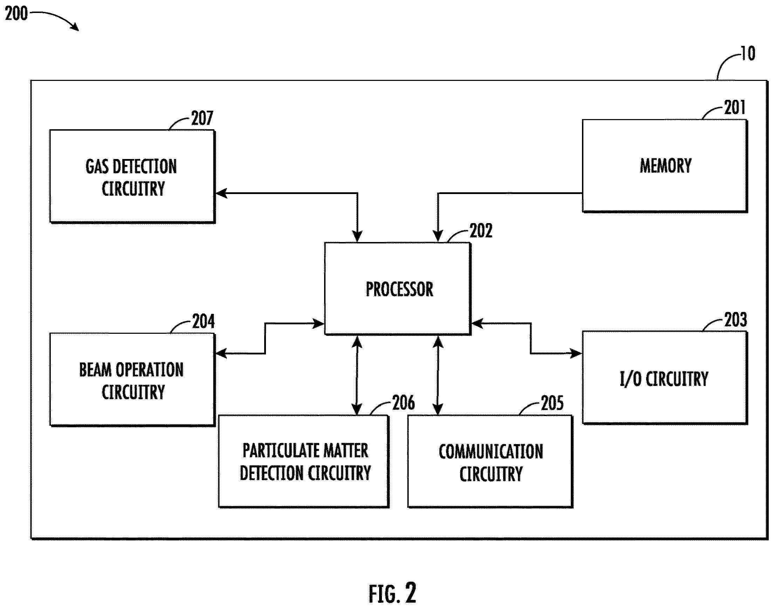

FIG. 2 illustrates a schematic view of an exemplary apparatus in accordance with various embodiments described herein. In particular, FIG. 2 illustrates a schematic view of a controller 200 of an exemplary sensor 10, in accordance with various embodiments of the present disclosure. In various embodiments, as illustrated in FIG. 2, an exemplary controller 200 may comprise a memory 201, a processor 202, input/output circuitry 203, communication circuitry 205, particulate matter detection circuitry 206, gas detection circuitry 207, and beam operation circuitry 204. The controller 200 may be configured to execute the operations described herein. Although the components are described with respect to functional limitations, it should be understood that the particular implementations necessarily include the use of particular hardware. It should also be understood that certain of the components described herein may include similar or common hardware. For example, two sets of circuitry may both leverage use of the same processor, network interface, storage medium, or the like to perform their associated functions, such that duplicate hardware is not required for each set of circuitry. The use of the term "circuitry" as used herein with respect to components of the controller 200 should therefore be understood to include particular hardware configured to perform the functions associated with the particular circuitry as described herein.

The term "circuitry" should be understood broadly to include hardware and, in some embodiments, software for configuring the hardware. For example, in some embodiments, "circuitry" may include processing circuitry, storage media, network interfaces, input/output devices, and the like. In some embodiments, other elements of the controller 200 may provide or supplement the functionality of particular circuitry. For example, the processor 202 may provide processing functionality, the memory 201 may provide storage functionality, the communications circuitry 205 may provide network interface functionality, and the like.

In some embodiments, the processor 202 (and/or co-processor or any other processing circuitry assisting or otherwise associated with the processor) may be in communication with the memory 201 via a bus for passing information among components of the apparatus. The memory 201 may be non-transitory and may include, for example, one or more volatile and/or non-volatile memories. For example, the memory 201 may be an electronic storage device (e.g., a computer readable storage medium). In various embodiments, the memory 201 may be configured to store information, data, content, applications, instructions, or the like, for enabling the apparatus to carry out various functions in accordance with example embodiments of the present disclosure. It will be understood that the memory 201 may be configured to store partially or wholly any electronic information, data, data structures, embodiments, examples, figures, processes, operations, techniques, algorithms, instructions, systems, apparatuses, methods, look-up tables, or computer program products described herein, or any combination thereof. As a non-limiting example, the memory 201 may be configured to store timestamp data, location data, and/or the like, as well as gas data, such as, for example, at least a portion of a frequency-comb data stream, gas concentration data, gas property data, and/or the like associated with a volume of gas. As a further non-limiting example, the memory 201 may be configured to store particulate data, such as, for example, particulate size data, particulate type data, particulate shape data, particulate cross-sectional area data, particulate mass data, particulate matter concentration data, particulate quantity data and/or the like, associated with a volume of gas.

The processor 202 may be embodied in a number of different ways and may, for example, include one or more processing devices configured to perform independently. Additionally or alternatively, the processor may include one or more processors configured in tandem via a bus to enable independent execution of instructions, pipelining, and/or multithreading. The use of the term "processing circuitry" may be understood to include a single core processor, a multi-core processor, multiple processors internal to the apparatus, and/or remote or "cloud" processors.

In an example embodiment, the processor 202 may be configured to execute instructions stored in the memory 201 or otherwise accessible to the processor. Alternatively, or additionally, the processor may be configured to execute hard-coded functionality. As such, whether configured by hardware or software methods, or by a combination thereof, the processor may represent an entity (e.g., physically embodied in circuitry) capable of performing operations according to an embodiment of the present disclosure while configured accordingly. Alternatively, as another example, when the processor is embodied as an executor of software instructions, the instructions may specifically configure the processor to perform the algorithms and/or operations described herein when the instructions are executed.

In some embodiments, the controller 200 may include input-output circuitry 203 that may, in turn, be in communication with the processor 202 to provide output to the user and, in some embodiments, to receive input such as a command provided by the user. The input-output circuitry 203 may comprise a user interface, such as a graphical user interface (GUI), and may include a display that may include a web user interface, a GUI application, a mobile application, a client device, or any other suitable hardware or software. In some embodiments, the input-output circuitry 203 may also include a display device, a display screen, user input elements, such as a touch screen, touch areas, soft keys, a keyboard, a mouse, a microphone, a speaker (e.g., a buzzer), a light emitting device (e.g., a red light emitting diode (LED), a green LED, a blue LED, a white LED, an infrared (IR) LED, or a combination thereof), or other input-output mechanisms. The processor 202, input-output circuitry 203 (which may utilize the processing circuitry), or both may be configured to control one or more functions of one or more user interface elements through computer-executable program code instructions (e.g., software, firmware) stored in a non-transitory computer-readable storage medium (e.g., memory 201). Input-output circuitry 203 is optional and, in some embodiments, the controller 200 may not include input-output circuitry. For example, where the controller 200 does not interact directly with the user, the controller 200 may generate user interface data for display by one or more other devices with which one or more users directly interact and transmit the generated user interface data to one or more of those devices. For example, the controller 200, using user interface circuitry may generate user interface data for display by one or more display devices and transmit the generated user interface data to those display devices.

The communications circuitry 205 may be a device or circuitry embodied in either hardware or a combination of hardware and software that is configured to receive and/or transmit data from/to a network and/or any other device, circuitry, or module in communication with the apparatus 200. For example, the communications circuitry 205 may be configured to communicate with one or more computing devices via wired (e.g., USB) or wireless (e.g., Bluetooth, Wi-Fi, cellular, and/or the like) communication protocols. For example, as described in further detail herein, the communications circuitry 205 may be configured to facilitate communication between an exemplary fluid flow device and one or more computing devices of an exemplary fluid flow management system via wired (e.g., USB, ethernet, and/or the like) or wireless (e.g., Bluetooth, Wi-Fi, cellular, and/or the like) communication protocols, as described in further detail herein.

In various embodiments, the processor 202 may be configured to communicate with the particulate matter detection circuitry 206. The particulate matter detection circuitry 206 may be a device and/or circuitry embodied in either hardware or a combination of hardware and software that is configured to receive, process, generate, and/or transmit data (e.g., particulate data), such as a photodetector signal generated by a photodetector arranged within the sensor 10. In various embodiments, the particulate matter detection circuitry 206 may be configured to detect the presence of particulate matter within the sensing region of the sensor 10 (e.g., at one or more locations along the beam path of the beam emitted from the beam component) based at least in part on the particulate data received from the one or more photodetectors of the sensor 10.

Further, in various embodiments, the particulate matter detection circuitry 206 may be configured to determine a particulate matter concentration associated with detected particulate matter within a volume of gas based at least in part on the particulate data (e.g., a photodetector signal) received from one or more of the photodetectors of the sensor 10. For example, in various embodiments, the particulate matter detection circuitry 206 may determine a particulate matter concentration as defined by the one or more particulates detected by a photodetector based at least in part on the pulse quantity and/or a pulse intensity defined by the portion of the optical beam detected by the photodetector.

Further, in various embodiments, the particulate matter detection circuitry 206 may be configured to execute a data processing operation wherein a photodetector signal received from the one or more photodetectors and defined at least in part by the particulate data generated by the one or more photodetectors is used together with a known optical pulse rate defined by an operation of the beam component (e.g., the light beam generator) in a phase-locked loop (PLL) to enhance the photodetector signal. For example, particulate data generated by one or more photodetector of the sensor 10 may be received by the particulate matter detection circuitry 206 and processed with the optical pulse rate using a phase-locked loop to at least partially reduce and/or minimize the background noise within the photodetector signal captured by the photodetector.

In various embodiments, the processor 202 may be configured to communicate with the gas detection circuitry 207. The gas detection circuitry 207 may be a device or circuitry embodied in either hardware or a combination of hardware and software that is configured to receive, process, generate, and/or transmit data (e.g., gas data) associated with a gas sensing operation executed by the sensor 10. For example, in various embodiments, the gas detection circuitry 207 may be configured to receive gas data generated by a beam component of the sensor 10, such as, for example, a frequency-comb data stream that includes multiple wavelengths. In various embodiments, the gas detection circuitry 207 may detect the presence of a specific gas within a sensing region based at least in part on the gas data generated by the beam component via an optical gas sensing means. For example, in various embodiments, the gas detection circuitry 207 may be configured to execute one or more instructions defining a dual-frequency-comb spectroscopy method of detecting one or more pre-designated gases within a sensing region.

Further, in various embodiments, the gas detection circuitry 207 may be configured to determine a gas concentration associated with detected gas within a sensing region based at least in part on the gas data received from the beam component of the sensor 10. For example, in various embodiments, the gas detection circuitry 206 may determine a gas concentration of a specific gas based at least in part on an absorption intensity defined at specific wavelength defined within the known absorption band of the specific gas.

In various embodiments, the processor 202 may be configured to communicate with the beam operation circuitry 204. The beam operation circuitry 204 may be a device or circuitry embodied in either hardware or a combination of hardware and software that is configured to selectively control operation of the beam component (e.g., the light beam generator) of the sensor 10 as part of one or more gas and/or particulate matter sensing operations. In various embodiments, the beam operation circuitry 204 may be configured to generate a control signal for transmission to the beam component that is configured to cause the beam component to emit an optical beam therefrom according to one or more predefined characteristics, such as, for example, a predetermined optical pulse rate.

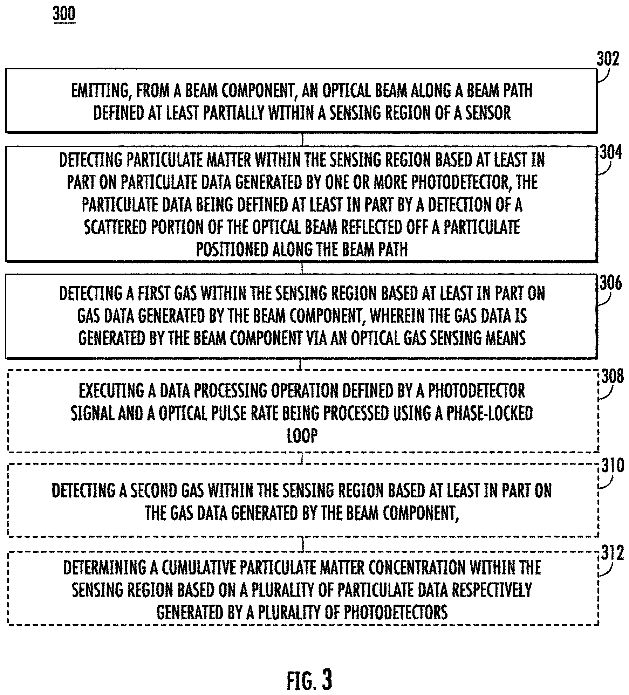

FIG. 3 is an illustrative flowchart of various steps for an example method in accordance with various embodiments of the present disclosure. In particular, FIG. 3 a flowchart of an exemplary method 300 of detecting one or more gases and particulate matter within a sensor is provided. In some embodiments, one or more operations of the illustrated exemplary method 300 may be executed by controlling a sensor in accordance with one or more example embodiments described herein. For example, various operations discussed below with respect to exemplary method 300 may be carried out using various components of an exemplary sensor, such as, for example, an exemplary sensor 10 as described above in reference to FIGS. 1-2.

As illustrated in FIG. 3, exemplary method 300, at Block 302, may include emitting, from a beam component, an optical beam along a beam path defined at least partially within a sensing region of a sensor. In various embodiments, emitting the optical beam from the beam component may include pulsing the optical beam such that the optical beam defines an active optical beam defined at least in part by an optical pulse rate.

Further, at Block 304, particulate matter may be detected within the sensing region based at least in part on particulate data generated by one or more photodetector. As described herein, the particulate data generated by the one or more photodetector may be defined at least in part by a detection of a scattered portion of the optical beam that reflects off a particulate positioned along the beam path. For example, a scattered portion of the optical beam that reflects off a particulate positioned along the beam path adjacent (e.g., within a field of view of) a photodetector may be detected by the photodetector and, in response, particulate data associated with the particulate may be generated (e.g., by the photodetector) to facilitate a determination of one or more particulate characteristics (e.g., particulate size, particulate matter concentration, and/or the like) defined by the particulate. In various embodiments, detecting the particulate matter within the sensing region may be defined at least in part by an operation of determining (e.g., via a controller) a particulate matter concentration associated with the particulate matter based at least in part on the particulate data. For example, the particulate data may be transmitted by a photodetector to a controller configured to execute at least a portion of the particulate matter sensing operation steps described herein.

Further, at Block 306, a first gas may be detected within the sensing region based at least in part on gas data generated by the beam component. As described herein, the gas data may be generated by the beam component via an optical gas sensing means. In various embodiments, the gas data may be defined at least in part by a detected absorption (e.g., an absorption intensity) of at least a portion of the optical beam emitted from the beam component at a first wavelength corresponding to the first gas. For example, dual-frequency-comb spectroscopy may be used to detect the presence of a first gas based at least in part on a portion of the generated gas data (e.g., a frequency-comb data stream including multiple wavelengths) defined at a first wavelength that is within the absorption band of the first gas. In various embodiments, detecting the first gas may be defined at least in part by an operation of determining (e.g., via a controller) a gas concentration associated with the first gas based at least in part on the gas data. For example, the gas data may be transmitted by a beam component to a controller configured to execute at least a portion of the gas sensing operation steps described herein. In various embodiments, the gas data generated by the beam component may be further defined by an absorption intensity defined at the first wavelength. For example, in such an exemplary circumstance, the gas concentration associated with the first gas may be determined based at least in part on the absorption intensity defined at least at the first wavelength, as measured by the beam component.

In various embodiments, as illustrated at Block 308, an exemplary method 300 of detecting one or more gases and particulate matter within a sensor may further include executing a data processing operation wherein a photodetector signal defined at least in part by the particulate data generated by the one or more photodetectors is processed with the optical pulse rate using a phase-locked loop to enhance the photodetector signal by at least partially reducing signal noise.

In various embodiments, as illustrated at Block 310, an exemplary method 300 of detecting one or more gases and particulate matter within a sensor may further include detecting a second gas within the sensing region based at least in part on gas data generated by the beam component. For example, dual-frequency-comb spectroscopy may be used to detect the presence of the second gas based at least in part on a portion of the generated gas data (e.g., the frequency-comb data stream including multiple wavelengths) defined at a second wavelength that is within the absorption band of the second gas.

Further, in various embodiments, as illustrated at Block 312, an exemplary method 300 of detecting one or more gases and particulate matter within a sensor may include determining a cumulative particulate matter concentration within the sensing region based on a plurality of particulate data respectively generated by a plurality of photodetectors. For example, respective particulate data generated by each of a plurality of photodetectors may be received (e.g., by a controller) from the plurality of photodetectors and used to determine the cumulative particulate matter concentration. In various embodiments, the cumulative particulate matter concentration may be defined at least in part by a plurality of particulates positioned at respective locations within the sensing region (e.g., along respective portions of the beam path) that are detected by the photodetectors via a corresponding plurality of optical beam reflections captured by the photodetectors.

As a non-limiting example provided for illustrative purposes, a first scattered portion of the optical beam reflected off a first particulate positioned along the beam path adjacent (e.g., within a field of view of) a first photodetector may be detected by the first photodetector, a second scattered portion of the optical beam reflected off a second particulate positioned along the beam path adjacent a second photodetector may be detected by the second photodetector, a third scattered portion of the optical beam reflected off a third particulate positioned along the beam path adjacent a third photodetector may be detected by the third photodetector, and a fourth scattered portion of the optical beam reflected off a fourth particulate positioned along the beam path adjacent a fourth photodetector may be detected by the fourth photodetector. In such an exemplary circumstance, in response to the first, second, third, and fourth scattered portions of the optical beam being detected by the first, second, third, and fourth photodetectors, respectively, the first, second, third, and fourth photodetectors may respectively generate first, second, third, and fourth particulate data associated with the corresponding first, second, third, and fourth particulate adjacent thereto. The cumulative particulate matter concentration defined by the particulate matter within the sensing region (e.g., the first, second, third, and fourth particulates) a may be determined (e.g., by a controller) based in part on each of the first, second, third, and fourth particulate data generated by the respective photodetectors of the plurality. Further, in various embodiments, the particulate matter concentration along the beam path may be at least partially spatially resolved based at least in part on the first, second, third, and fourth particulate data generated by the plurality of photodetectors.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A method of detecting one or more gases and particulate matter within a sensing region, the method comprising:
emitting, from a beam component, an optical beam along a beam path defined at least partially within a sensing region;
detecting particulate matter within the sensing region based at least in part on particulate data generated by one or more photodetector, the particulate data being defined at least in part by a detection of a scattered portion of the optical beam reflected from a particulate positioned along the beam path; and
detecting a first gas within the sensing region based at least in part on gas data generated by the beam component, wherein the gas data is generated by the beam component via an optical gas sensing means, and wherein the gas data is defined at least in part by a detected absorption of at least a portion of the optical beam at a first wavelength corresponding to the first gas.

2. The method of claim 1, wherein the optical beam embodies an active optical beam that is pulsed from the beam component such that the active optical beam defines an optical pulse rate.

3. The method of claim 2, further comprising executing a data processing operation wherein a photodetector signal defined at least in part by the particulate data generated by the one or more photodetectors is processed with the optical pulse rate using a phase-locked loop to enhance the photodetector signal by at least partially reducing signal noise.

4. The method of claim 1, wherein the optical gas sensing means is a dual-frequency comb spectroscopy operation.

5. The method of claim 4, wherein the gas data generated by the beam component comprises a frequency-comb data stream including at least a first wavelength and a second wavelength.

6. The method of claim 1, further comprising providing a color filter relative to the one or more photodetectors such that the scattered portion of the optical beam is passed through the color filter.

7. The method of claim 1, further comprising transmitting the particulate data generated by the one or more photodetector to a controller, and determining, via the controller, a particulate matter concentration associated with the particulate matter based at least in part on the particulate data.

8. The method of claim 1, further comprising transmitting the gas data generated by the beam component to a controller, and determining, via the controller, a gas concentration associated with the first gas based at least in part on the gas data.

9. The method of claim 8, wherein the gas data is further defined by an absorption intensity defined at the first wavelength, and wherein the gas concentration associated with the first gas is determined based at least in part on the absorption intensity defined at least at the first wavelength.

10. The method of claim 1, wherein the one or more photodetectors defines a plurality of photodetectors, and wherein the method further comprises determining a cumulative particulate matter concentration based on the respective particulate data generated by each of the plurality of photodetectors.

11. The method of claim 10, wherein the beam path defined by the optical beam defines a looped path configuration, wherein the beam path is defined along two or more distinct linear axes.

12. A sensor configured for detecting one or more gases and particulate matter, the sensor comprising:
a beam component configured to emit an optical beam along a beam path defined at least partially within a sensing region of a sensor, wherein the beam component is further configured to generate gas data associated with a first gas positioned the sensing region via an optical gas sensing means;
one or more photodetector configured to generate particulate data defined at least in part by a detection of a scattered portion of the optical beam reflected off a particulate positioned along the beam path; and
a controller configured to detect particulate matter within sensing region based at least in part on the particulate data generated by the one or more photodetector, and detect the first gas based at least in part on the gas data generated by the beam component, wherein the gas data is defined at least in part by a detected absorption of at least a portion of the optical beam at a first wavelength corresponding to the first gas.

13. The sensor of claim 12, wherein the optical beam embodies an active optical beam that is pulsed from the beam component such that the active optical beam defines an optical pulse rate.

14. The sensor of claim 12, wherein the optical gas sensing means is a dual-frequency comb spectroscopy operation.

15. The sensor of claim 12, wherein the one or more photodetectors defines a plurality of photodetectors each oriented to face in a direction at least substantially towards a respective portion of the beam path and configured to generate respective particulate data defined at least in part by respective detections of scattered optical beam light reflected from one or more particulates positioned at the respective portion of the beam path adjacent the respective photodetector.
